# EUROPEAN PATENT APPLICATION

(11) **EP 1 832 604 A1**
(43) Date of publication of application: **12.09.2007**
(21) Application number: 06004944.2
(22) Date of filing: 10.03.2006
(51) Int. Cl.: C07K 14/47, A61K 39/00

(54) **Use of IEX-1 for the treatment of glioma tumors**

(71) Applicant: TIMA Foundation, 9496 Balzers (LI)
(72) Inventor: Graf Matuschka von Greiffenclau, Markus, 9220 Bischofszell (CH)
(74) Representative: Dehmel, Albrecht

(57) **Abstract**

The present invention relates to a nucleic acid molecule encoding for IEX-1 polypeptide as a medicament. In a further aspect the present invention relates to a nucleic acid molecule encoding for IEX-1 polypeptide for the manufacture of a medicament for the treatment of gliomas.

## Description

The present invention relates to a nucleic acid molecule encoding for IEX-1 polypeptide as a medicament. In a further aspect the present invention relates to a nucleic acid molecule encoding for IEX-1 polypeptide for the manufacture of a medicament for the treatment of gliomas.

Malignant gliomas are the most common malignant brain tumors. Gliomas are neuronal malignancies that originate from an uncontrolled proliferating cell of the central nervous system. Patients with a diagnosis of glioma have a dismal prognosis with an average five-year survival rate of less than 5%. The symptoms vary with the site of the tumor but tend to develop very quickly due to the rapid growth behaviour of the tumor cell. Among the first symptoms can be headaches, epileptic seizures, paralysis or changes of the personality of the patient.

Gliomas can originate from several cell types including ependymal cells, astrocytes, oligodendrocytes and different types of glia cells. Clinically, gliomas are divided into four grades, which are determined by pathologic evaluation of the tumor. Low grade gliomas are well-differentiated, slower growing, biologically less aggressive, and portend a better prognosis for the patient. High grade gliomas are undifferentiated or anaplastic, fast growing and can invade adjacent tissues, and carry a worse prognosis. Unfortunately, the most aggressive of these grades, grade 4 or glioblastoma multiforme (GBM), is also the most frequent in humans. Because most patients with GBMs die of their disease in less than a year and essentially no patient has long-term survival, these tumors have drawn significant attention; however, they have evaded successfully all attempts at therapy over the last half-century.

One of the reasons for the resistance of GBM to therapeutic treatments is the complex character of the tumor itself. As the name GBM implies, glioblastoma is multiforme. It is multiforme grossly, showing regions of necrosis and hemorrhage. It is multiforme microscopically, with regions of pseudopalisading necrosis, pleomorphic nuclei and cells, and microvascular proliferation. And it is genetically diverse, with various deletions, amplifications, and point mutations leading to activation of signal transduction pathways downstream of tyrosine kinase receptors such as epidermal growth factor receptor (EGFR) and platelet-derived growth factor receptor (PDGFR), as well as to disruption of cell-cycle arrest pathways by INK4a-ARF loss or by p53 mutations associated with cyclin-dependent kinase 4(CDK4) amplification or retinoblastoma-protein (Rb) loss.

One of the main reasons that the gliomas are not cured by surgery is the topographically diffuse nature of the disease. In addition to the above-mentioned variability within the tumor proper, the location of the tumor cells within the brain also is variable, resulting in the inability to completely resect this tumor. Glioma cells migrate away from the initial tumor through the brain parenchyma, collect just below the pial margin (subpial spread), surround neurons and vessels (perineuronal and perivascular satellitosis), and migrate through the white matter tracks (intrafacicular spread). The final outcome of this behavior is the spread of individual tumor cells diffusely over long distances and into areas of the brain which are essential for the survival of the patient. The extreme example of this behavior is a condition referred to as gliomatosis cerebri, in which the entire brain is diffusely infiltrated by neoplastic cells with minimal or no. central focal area of tumor per se. Gliomas do thus not metastasize by the bloodstream, but they can spread via the cerebrospinal fluid and cause "drop metastases" to the spinal cord. 25% of patients with GBM have multiple or multi centric GBMs at autopsy. Therefore, the infiltrative growth pattern of these tumors precludes curative neurosurgery and high grade gliomas almost always grow back even after "complete" surgical excision. Even with repeat surgeries for tumor recurrences, the patients eventually die from tumor spread into vital regions of the brain.

Due to the complex behaviour of glioma malignancies the standard treatment of high grade gliomas can not rely only on a single specific method and treatment strategy, respectively. The therapy has been essentially the same for many years: a combination of surgical resection of as much of the tumor as feasible, followed by radiation therapy and chemotherapy (usually designed to damage DNA or to otherwise inhibit DNA replication). But even under the best of circumstances, in which essentially all of the tumor (which can be monitored via a MRI scan) can be surgically removed and the patients are fully treated with radiation and chemotherapy, the mean survival of this disease is just extended from 2 to 3 months to 1 year. Very often, the tumor is a mixture of cells with and without functional (wild-type) tumor-suppressive gene p53 product, which affects the sensitivity to irradiation or chemotherapy. While a functional wild-type p53 gene enhances the sensitivity to chemotherapy it functions as a resistant factor to irradiation. The same applies vice versa.

The poor outcome of the standard treatments for GBM in combination with the diffuse nature of the disease lead to a number of attempts at novel therapeutic approaches with the aim of also killing neoplastic cells disseminated from the main tumor. Essentially these approaches comprise three strategies: an immunological approach, a gene therapeutic approach or a viral approach.

The immunological approach, an endeavour to entice the immune system to reject the tumor in a physiological fashion, has been investigated extensively with many successes in laboratory animals. However, translation of success in rodents to humans has not occurred. The malignancy of gliomas results not only from enhanced proliferation rates but also from a reduced level of physiological apoptosis. Thus, the tumor cells are usually not only resistant to irradiation and chemotherapy but also to immune therapy.

The more recent viral approach comprises the use of viral vectors that replicate specifically in tumor cells and, thereby, kill them lytically. These approaches utilize viruses that normally infect the central nervous system, but which have been modified for this application. The recombinant viruses are engineered in such a way, that they become non-pathogenic to normal tissues but remain lytic to neoplastic cells. Attenuated non-neurovirulent versions of herpes simplex virus have been used previously and have been shown to kill glioma cells in culture and implanted in rodents. Another example for this approach has been described by Gromeier et al. (PNAS 97, 2000: p. 6803-6808), who report the use of a hybrid virus (polio virus-human rhinovirus) that does not have the neurovirulence of polio virus but does infect and kill clonal human glioma cell lines both in culture and as xenographs in athymic mice. Some of these viruses are currently in clinical trials.

The gene therapy approach relies on the transfer of lethal genes to tumor cells. The classic example for this strategy was a retroviral transfer of the herpes simplex virus thymidine kinase (TK) gene to tumor cells followed by treatment with the antiviral compound gancyclovir to kill cells expressing TK. Preliminary reports showed that this strategy was capable to eradicate experimentally implanted gliomas in rodents. Unfortunately, the application of this strategy to human gliomas seems not to have a therapeutic benefit in humans.

It is obvious, that for the treatment of glioma diseases there is currently no strategy available, which provides proper chances of success, i.e. to cure the patient. In this respect, even the combination of all currently applied treatments seems not to be sufficient.

The human immediate early gene X-1 (IEX-1, also known as Difl and PRG1), the human ortholog of murine gly96 was initially identified in human squamous carcinoma cells as an X-radiation-inducible immediate early gene. The IEX-1 gene encodes a protein of 156-amino acids and shares no significant sequence similarities with any other known protein. The protein undergoes post-translational glycosylation to yield a product of 27-29 kDa. In spite of lacking significant sequence homologies with other known proteins, human, rat and mouse IEX-1 are well conserved (>73%) in the amino acid sequence.

IEX-1 transcription is under the control of transcription factors like p53, c-Myc and NF-κB/rel that have been documented to be capable of manipulating mutually exclusive processes, for instance, cell survival vs. cell death or cell division vs. cell cycle arrest, depending on the cell types and the number and strength of stimuli. Coincidentally, like these transcription factors, IEX-1 can also positively and negatively affect cell survival and cell cycle progression. IEX-1 generates cellular signals implicated in proliferation, death, and survival signalling pathways. However, the exact molecular mechanisms of IEX-1 mediated cellular response are not fully disclosed yet. Immunochemical staining shows that IEX-1 protein is strongly expressed in epithelia of the skin, trachea, gastrointestinal, and genitourinary systems as well as in the pancreas and breasts. This pattern of tissue distribution suggests a role for IEX-1 in response to the external environment with which epithelial tissues make direct contact.

Several studies have shown that overexpression of IEX-1 facilitates apoptosis in response to certain stimuli, as evidenced by substantial increases in apoptotic cells and in caspase-3 activity in the presence compared to absence of ectopic IEX-1.

However, in addition to sensitization of apoptosis, IEX-1 may also have a role in the cell cycle regulation, because overexpression of IEX-1 is able to significantly promote cell cycle progression in HeLa and keratinocytes. This is consistent with the notion that cells proceeding in the cell cycle are prone to apoptosis, whereas cells residing in G1 arrest are more refractory.

Furthermore, in contrast to the in vitro studies utilizing cell lines, in vivo studies demonstrated that IEX-1 protects activated T cells from apoptosis. IEX-1 transgenic animals developed with an significantly increased probability splenomegaly, lymphadenopathy and lymphomas. Thus, IEX-1 expression has in vivo substantial oncogenic potential.

Additionally, high levels of IEX-1 expression are observed in many tumor cell lines. Consistent with this is the finding that IEX-1 expression can be suppressed by p53 through more than one mechanism. Therefore, inactivation of p53 (which is common in tumor cells) has been proposed to up-regulate IEX-1 expression, resulting in a predisposition to tumorigenesis by its effects on either cell cycle progression or cell survival. This also implicates, that IEX-1, whose expression is induced by radiation and many chemotherapeutic drugs at relatively high levels, might contribute to resistance of cancers to radiation and chemotherapy.

However, the exact role of IEX-1 in different cell types is not understood yet.

Therefore, it is an object of the present invention to provide an approach for the treatment of glioma and other related neuronal malignancies that enhances the survival rate.

The object is solved by the subject-matter as defined in the claims.

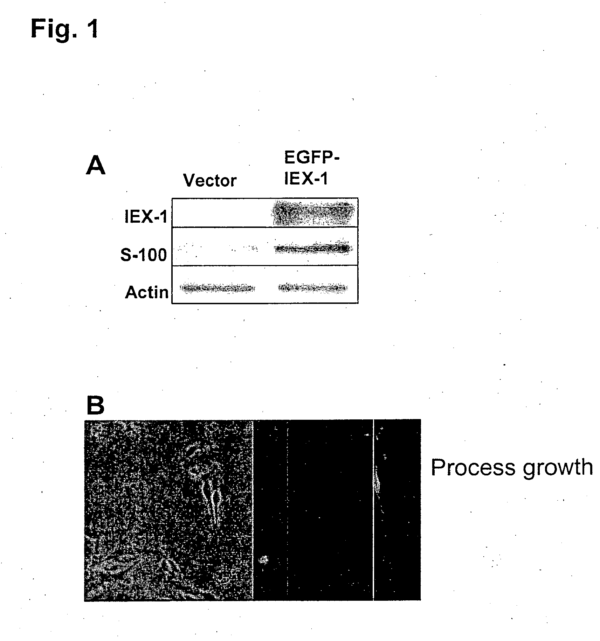

Figure 1: Transient transfection of U87MG p53(+) cells
   U87-MG cells were transiently transfected with pIRES2-EGFP plasmid vector containing IEX-1 cDNA (EGFP-IEX-1) for 24 h. A: The extracted proteins were subjected to Western blot analysis with anti-IEX-1, S-100 and actin antibodies. B: Cell morphology was analyzed by a phase contrast microscope (a) and GFP fluorescence of the same scene was by a fluorescent microscope (b). The results shown are representatives from at least three experiments.

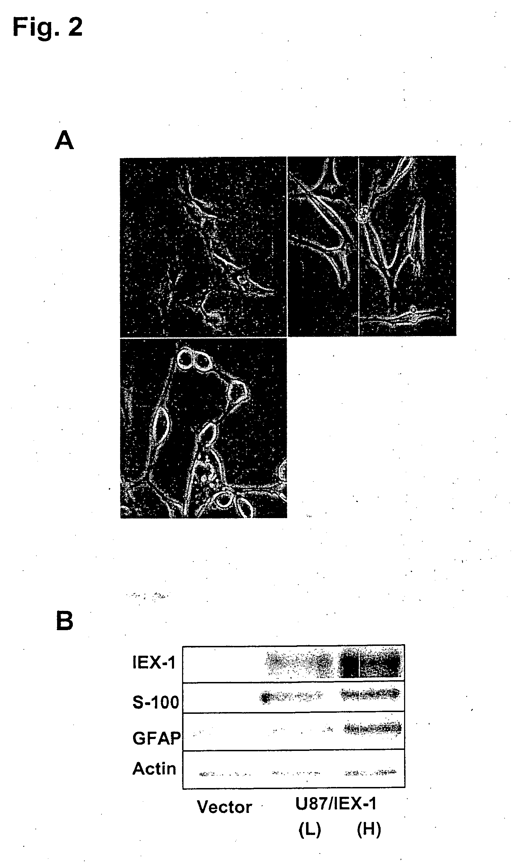

Figure 2: Stable transfection of U87MG p53(+) cells
   U87-MG cells were transfected with pIRES1neo vector containing IEX-1 cDNA. After selection with G418 (0.5mg/ml), the colonies expressing two different levels (H; high and L; low) of IEX-1 were obtained. A: Phase-contrast micrographs of cells. B: The extracted proteins were subjected to Western blot analysis with anti-IEX-1, S-100, GFAP, and actin antibodies. The results shown are representatives from at least three experiments.

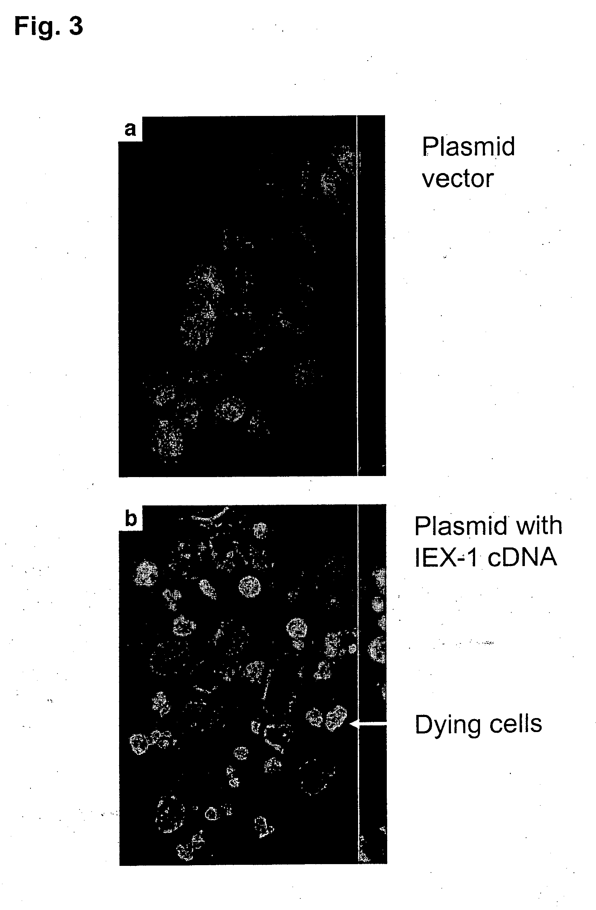

Figure 3: Transient transfection of U373 p53(-) cells
   U373 cells were transiently transfected with pIRES2-EGFP plasmid vector containing IEX-1 cDNA (EGFP-IEX-1) for 48 h. Apoptotic cells stained with Hoechst 33258 were quantified by fluorescent microscopic analysis. Briefly, cells were fixed in 1% glutaraldehyde for 30 min. The cells were then stained with 10 µM Hoechst 33258 for 10 min. Nuclear morphology was observed under a fluorescent microscope (Olympus BX60, Tokyo, Japan).

The present invention relates to a nucleic acid molecule comprising a sequence encoding an IEX-1 polypeptide for use as a medicament.

In particular, the present invention relates to the use of a nucleic acid molecule comprising a sequence encoding for an IEX-1 polypeptide, for the manufacture of a medicament for the treatment of gliomas.

The inventors surprisingly found, that the expression of human IEX-1 by a recombinant plasmid or vector in malignant glioma cells on its own allows the selective killing and/or inactivation of these glioma cells, while neuronal cells expressing the same construct remain unaffected by IEX-1 expression.

The finding that the recombinant expression of IEX-1 in malignant glioma cells allows the selective killing and/or inactivation of these glioma cells, and has thus anti-cancer properties in these regard, was unpredictable and unexpected and provides a new means to treat glioma and related conditions.

The term glioma, as used herein, relates to tumors, which arise from non-neuronal cells of the central nervous system, in particular glial and related cells, even more particular from ependymal cells, astrocytes, oligodendrocytes or other types of glial cells. Accordingly, the term glioma comprises disease conditions such as ependymomas, astrocytomas, in particular glioblastoma, oligodendrogliomas, oligoastrocytomas, and other gliomas of mixed origin, i.e. originating from more than one type of cell, including cells of astrocytes, ependymal cells and/or oligodendrocytes. The affected tissue can be of any portion of the nervous system, i.e. brain tissue as well as spinal cord tissue.

In a preferred embodiment, the present invention relates to a nucleic acid molecule for use in the present invention, wherein the nucleic acid molecule encoding IEX-1 comprises the nucleic acid sequence of SEQ ID NO: 1 or fragments thereof. In another preferred embodiment the IEX-1 polypeptide comprises the sequence of SEQ ID NO: 2. Alternatively, the nucleic acid molecule encoding for IEX-1 polypeptide can comprise the genomic sequence of the IEX-1 locus, i.e. can comprise additional to the coding sequence non-coding sequences of the genomic locus, for example in order to improve the expression of the polypeptide in the target cells.

Preferably, the nucleic acid molecule for use in the present invention is a vector functionally active in mammalian cells such as a viral vector, in particular a vector derived from adenoviruses, adeno-associated virus, herpes viruses, retroviruses or other mammalian viruses. In the state of the art a multitude of such expression vectors or means of construction thereof has been described and a person skilled in the art will find several ways to construct such vectors.

In one embodiment of the present invention the expression of the nucleic acid molecule encoding for IEX-1 polypeptide for use in the present invention is driven by a promoter, in particular by a promoter selected from SV40, CMV, thymidine kinase, E2A, LTR or wherein said promoter is an exogenously inducible promoter.

In an other preferred embodiment the expression or overexpression of the IEX-1 polypeptide is restricted to certain tissue and/or cell types by controlling its expression via a tissue and/or cell type specific promoter or by using a tumor specific promoter and/or by using appropriate enhancer cassettes up- or downregulating the transcriptional promoter activity in the tissue/cell type of interest. Preferably the tissue is part of the central nervous system and the cell type is glial. Such restrictions of expression are desirable in order to minimize adverse effects resulting from expression of IEX-1 in healthy tissues/cells. Examples for such promoters are, but are not restricted to, the human glial fibrillary acidic protein promoter (GFAP promoter) or the human telomerase reverse transcriptase promoter (hTERT promoter). GFAP is an intermediate filament protein expressed almost exclusively in astrocytes and frequently in gliomas (McLendon and Bigner, Brain Pathol. 4, 1994: p. 221-228). The GFAP promoter has been used successfully for expressing HSV-TK and gas1 in glioma cells (Vandier et al., Cancer Gene Ther. 7, 2000: p. 1120-1126; Zamorano et al., Neurobiol. Disease 15, 2004: p. 483-491). In respect of the hTERT promoter, approximately 90% of tumors have telomerase activity, whereas most of normal cells do not express telomerase. Telomerase activity is detected in 75~90 of glioblastoma multiforme (grade IV). In contrast, normal brain tissues do not express telomerase activity. hTERT promoter successfully worked in glioma cells to express caspase-6 (Komata et al., Cancer Res. 61, 2001: p. 5796-5802) and PUMA (Ito et al., Human Gene Ther. 16, 2005: p. 685-698). Thus, the present invention relates in an even more preferred embodiment to the use of an nucleic acid molecule according to the invention, which is driven by a promoter, which confers a tissue specific expression of said IEX-1 polypeptide in glial cells, in particular for example the GFAP-promotor mentioned above, or wherein said promoter confers a tumor specific expression, in particular the hTERT promoter. Preferably, the expression of recombinant IEX-1 is completely shut off in unrelated tissue/cells. The promoter can also be an inducible promoter, i.e. a promoter which becomes active or alternatively inactive only after exposure to certain agents or stimuli, such as the GAL promoter, tet regulatable promoter (tet on/off), and the like, as is known to everyone skilled in the art.

In a preferred embodiment according to the invention the medicament comprises additionally an inhibitor of p53 wild type protein. This inhibitor can be a nucleic acid molecule interfering with the expression of p53 or a nucleic acid molecule encoding for such a nucleic acid molecule interfering with the expression of p53 or any other molecule, which interferes with the expression of p53 gene product. A nucleic acid molecule interfering with p53 expression can be, for instance, a siRNA directed against p53 mRNA. An example for an other molecule interfering with expression of p53 is pifithrin-α. Other forms of inhibitors of p53 gene expression known to the skilled artisan can be applied as well. The co-administration of an inhibitor of p53 provides for an increase in efficient killing of the tumor cell, especially in cases, where the tumor has not lost wt p53

Where the glioma cell comprises a tumor in a patient, the vector is administered directly to the tumor or, in other embodiments, the vector is administered to the patient systemically or in a delivery vehicle containing a targeting moiety that directs delivery of the vector to the tumor, i.e. the glioma cells.

In a preferred embodiment the medicament is formulated in such a way, that it comprises a compound, which allows the transfer of the IEX-1 nucleic acid molecule to the target cells in a tissue or organism (shuttle). Such shuttle compounds are known to the skilled artisan and can be, for instance, liposomal compounds carrying on the outside of the liposomal membrane monoclonal antibodies (mAb), ligands and/or other proteins and substances, which recognize and bind specifically to certain cell surface markers of the target cell, i.e. providing for a specific tissue and/or cell targeting of the IEX-1 nucleic acid molecule, preferably an exclusive targeting to the glioma cell. Alternatively, specifically engineered viruses, e.g. Adenovirus, retrovirus, or virus based on HIV-capsid with appropriate epitopes can be applied. Adenoviral vectors provide for a transient transfection of the cell, as the DNA of adenoviruses is in contrast to retroviruses not integrated into the host genome and, thus, will be lost after several cell divisions. The use of adenoviruses is a preferred embodiment, if the target cell/target tumor tissue is only slowly or non-proliferating, as adenoviral infection is not dependent on proliferating cells. A retroviral approach is preferred in case of a rapidly growing tumor, because the target cells get permanently transfected.

The production of such viral particles or liposomes has been described in the prior art (Strauss M, et al., 1997; Tarhovsky YS et al., 1998; Anderson WF, 1998; Martin F et al., 1999; Kurane S et al., 1998).

Medicaments according to the invention can be employed in the form of pharmaceutical preparations. In such an embodiment the medicament comprises the nucleic acid molecule encoding for IEX-1 polypeptide and additionally a pharmaceutically acceptable carrier. Such preparations are made in a manner well known in the pharmaceutical art. One preferred preparation utilizes a vehicle of physiological saline solution, but it is contemplated that other pharmaceutically acceptable carriers such as physiological concentrations of other non-toxic salts, five percent aqueous glucose solution, sterile water or the like may also be used. It may also be desirable that a suitable buffer be present in the composition. Such solutions can, if desired, be lyophilized and stored in a sterile ampoule ready for reconstitution by the addition of sterile water for ready injection.

These pharmaceutical compositions can be administered to the subject at a suitable dose. Administration of the suitable compositions may be effected by different ways, e.g. by intravenous, intraperitoneal, subcutaneous, intramuscular, topical or intradermal administration.

In certain embodiments compositions containing IEX-1-encoding nucleic acid molecules are to be administered orally. Such formulations are preferably encapsulated and formulated with suitable carriers in solid dosage forms. Some examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, gelatin, syrup, methyl cellulose, methyl-and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated so as to provide rapid, sustained, or delayed release of the active ingredients after administration to the patient by employing procedures well known in the art. The formulations can also contain substances that diminish proteolytic degradation and promote absorption such as, for example, surface active agents.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Such excipients are those substances usually and customarily employed to formulate dosages for parenteral administration in either unit dosage or multidose form or for direct infusion into the cerebrospinal fluid by continuous or periodic infusion.

In another preferred embodiment the medicament according to the invention comprising a nucleic acid molecule encoding for IEX-1 polypeptide is formulated for a combined administration with an other tumor, i.e. second tumor therapy such as chemotherapy, radiation therapy, surgery, immune therapy, viral therapies or other gene therapeutic approaches relying on other lethal genes than IEX-1. The administration of the medicament according to the invention can be prior, concomitant or after administration of the second therapy.

The radiation therapy can be any form of radiation therapy used in the art such as for example, external beam radiation (e.g. X-ray or γ-ray treatment), radiation delivered by insertion of radioactive materials within the body near or at the tumor site (e.g. treatment with γ-ray emitting radionuclides), particle beam therapy which utilizes neutrons or charged particles and the like.

Another embodiment of the invention involves the use of the composition according to the invention in combination with chemotherapy. Chemotherapeutic agents are known in the art and include antimetabolites including pyrimidine-analogue and purine-analogue antimetabolites, plant alkaloids, antitumor antibiotics, alkylating agents and the like.

In one aspect, the present invention relates to a pharmaceutical composition comprising a nucleic acid molecule according the invention.

A further aspect of the invention is a method of treating a disease, in particular glioma, by administering a nucleic acid molecule encoding for an IEX-1 polypeptide according to the invention to a subject. In a preferred embodiment the method of treating glioma relates to gliomas selected from ependymomas, astrocytomas, in particular glioblastoma, oligodendrogliomas, oligoastrocytomas, or mixed gliomas originating from at least two types of glial cells. In a preferred embodiment the method of treatment according to the invention comprises the administering of a nucleic acid molecule encoding for IEX-1 polypeptide comprising the sequence of SEQ ID NO: 1 or SEQ ID NO: 2 to a subject. In a further preferred embodiment the method of treatment comprises the administering of a nucleic acid molecule to a subject, wherein the nucleic acid molecule is a vector, in particular a viral vector derived from adenoviruses, adeno-associated virus, herpes viruses, retroviruses, or other mammalian viruses. In another preferred embodiment the method of treatment comprises the administering of a nucleic acid molecule encoding for IEX-1 polypeptide to a subject, wherein the expression of the nucleic acid molecule is driven by a promoter, in particular by a promoter selected from SV40, CMV, thymidine kinase, E2A, LTR or wherein said promoter is an exogenously inducible promoter. The method of treatment can also comprise the administering of said nucleic acid molecule, wherein the promoter driving the expression of IEX-1 polypeptide confers a tissue specific expression or wherein said promoter confers a tumor specific expression. In particular, such a tissue specific promoter is the GFAP promoter or, in case of a tumor specific promoter, is the hTERT promotor. Preferably, the method of treatment allows for the combined administration or application of a second method of treatment such as chemotherapy, radiation therapy, surgery, immune therapy, viral therapies or other gene therapeutic approaches relying on other lethal genes than IEX-1. In a preferred embodiment the method of treatment according to the invention comprises additionally the administration of an inhibitor of p53.

Other embodiments within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims which follow the

### examples.

The invention will now be described in more detail by way of the following non-limiting

### examples.

### Example 1: Cloning of the IEX-1 cDNA containing plasmid vector

For expression of IEX-1, the full-length cDNA was amplified by PCR with the primers: sense, 5'-CGGAATTCCGATGTGTCACTCTCGCAGCTG-3' (SEQ ID NO: 3) and antisense, 5'-CGGAATTCCGTTAGAAGGCGGCCGGGTGTT-3' (SEQ ID NO: 4). The cDNA fragment was subcloned into a pCR-II-Topo vector and the nucleotide sequence was determined. The plasmid was digested with EcoRI, and the resulting IEX-1 cassette was subcloned into the EcoRl digested pIRES2-EGFP plasmid vector. The resulting vector was termed pIRES2-EGFP/IEX1. This vector provides now for the simultaneous expression of IEX-1 and EGFP.

### Example 2: Transfection of glioma cells with IEX-1 plasmid vector

The human glioblastoma cell lines U87-MG, U-373 MG and U251 MG were obtained from American Type Culture Collection (Rockville, MD, USA). The cells were maintained in Dulbecco's modified Eagle's medium (DMEM) supplemented with 10% (v/v) fetal bovine serum (FBS), 100 units/ml peniclillin and 100 µg/ml Streptomycin (FBS/DMEM) in a humidified atmosphere containing 5% CO₂ at 37°C. The expression plasmid was transfected into U-87 MG (p53+), U373 MG (p53-) and U251 MG (p53-) glioma cells using Trans-IT according to the manufacturer's instructions. As a control, PC12 neuronal cells were transfected as well. Cells transfected with pIRES2-EGFP/IEX1 plasmid vector express both green florescent protein (GFP) and IEX1. Therefore, successfully transfected cells are easily recognized by their green fluorescence.

### Example 3: Inactivation of functional p53

Functional p53 of U87 MG cells can be downregulated by retroviral gene transfer with HPV E6 protein, which binds p53 and accelerates its proteolytic degradation through the ubiquitin pathway. Therefore cells of the p53(+) cell line U-87 MG were transfected with a retroviral vector encoding for E6 protein. U87-LXSN (empty vector-infected), U87-W E6 (expressing wild-type E6) and U87-M E6 (expressing mutant-type E6) cell lines were obtained by infecting U-87 MG cells with LXSN, LXSN-16E6SD, and LXSN-16E6SD-8S9A10T retroviruses, respectively, followed by drug selection with 800 µg/ml G418 for 2 weeks (see Sawada et al. Oncogene 20, 2001: p. 1368-1378). Alternatively, p53 expression can also be targeted by siRNA using retroviral delivery of shRNA. To generate retrovirus vector expressing shRNA, the self-inactivating virus vector was used. U-87 MG cells expressing p53 shRNA (designed as U87-p53SI) and the control cells H1R (U87-Vec) were obtained by infection of SI-MSCVpuro-H1R-p53Ri and the backbone (SI-MSCVpuro-HIR) retroviruses, respectively, followed by selection with 1 µg/ml of puromycin. The 3' LTR in a murine stem cell virus vector, pCMSCVpuro-DEST was inactivated by an internal deletion *(Nhe*I*-Xba*I*).* The self-inactivating virus vector was named pSI-CMSCVpuro-DEST. The H1 promoter cassette with or without stuffer previously described by Brummelkamp et al. was attached by attB2 and attB1 sequences by adaptor PCR, and recombined into pDONR221 by BP reaction according to the manufacturer's instruction to generate pENTR221-H1R-stuffer or pENTR-H1R. To generate pENTR221-H1Rp53Ri, the pENTR221-H1R-stuffer was digested with *Bgl*II and *Hind*III and the annealed oligos (5'-gatccccGACTCCAGTGGTAATCTACttcaagagaGTAGATTACCACTGGAGTCttttttggaa a-3' (SEQ ID NO: 5) and 5'-agcttttccaaaaaGACTCCAGTGGTAATCTACtctcttgaaGTA GATTACCACTGGAGTCggg-3' (SEQ ID NO: 6) were replaced with the stuffer. Both inserts were recombined into pSI-CMSCVpuro-DEST by LR reaction according to the manufacture's instruction to generate pSI-CMSCVpuro-HIR-p53Ri and pSI-CMSCVpuro-H1R. U-87 MG cells expressing p53 shRNA (designed as U87-p53SI) and the control cells H1R (U87-Vec) were obtained by infection of SI-MSCVpuro-HIR-p53Ri and the backbone (SI-MSCVpuro-HIR) retroviruses, respectively, followed by selection with 1 µg/ml of puromycin.

### Example 4: Cell death analysis

Cell death analysis can be performed in two different ways. (1) Since pIRES2-EGFP contains a neomycin (Cr418)-resistant gene, cells, which did not receive pIRES2-EGFP/IEX1 plasmid, were first killed by 0.5 mg/ml G418 and then the number of surviving cells with green fluorescence was counted. (2) Duplicate culture dishes were transfected by either pIRES2-EGFP vector or pIRES2-EGFP/IEX1. After 72 hours, the number of cells with green fluorescence in each dish was compared. In some experiments, apoptotic cells stained with Hoechst 33258 were quantified by fluorescent microscopic analysis.

Glioma cells without wild type p53 expression levels, either completely absent or downregulated (p(53(-), U373 MG, U251 MG, U87-W E6, U87-p53SI) entered after expression of pIRES2-EGFP/IEX1 apoptosis and faced apoptotic cell death.

### Example 5: Analysis of non-glial cells expressing pIRES2-EGFP/IEX1

Cells of non-glial origin were transfected with pIRES2-EGFP/IEX1 and analysed as mentioned above. Shapes and viability of cultured non-neuronal cells (fibroblast NIH 3T3, chondrocyte HCS-2/8, HeLa, SCC14A squamous carcinoma cells) were not affected by expression of IEX-1cDNA. The same applied to neuronal but non-glial cells like PC12 cells (pheochromocytoma; neuronal cell model).

### Example 6: Detection of proteins by Western blot analysis

Total cellular protein extracts were used for the Western blot analysis. The cells were solubilized in ice-cold lysis buffer (50 mM Tris-HCI, pH 7.4, 150 mM NaCl, 10 mM EGTA, 1 % Triton-X, 0.1 % SDS, 1 % deoxycholic acid, 0.3 mM PMSF, 30 µg/ml (L-3-transcarboxyoxirane-2-carboryl)-L-leucyl-agmatine (E64), 1 mM sodium orthovanadate, 10 mM sodium fluoride, 0.1 mM sodium molybdate, 0.5 mM 4-deoxypyridoxine). The proteins were separated by SDS-PAGE, and were electrophoretically transferred onto polyvinylidene difluoride membranes. The membranes were first incubated with the indicated primary antibody, and then incubated with the anti-mouse or anti-goat IgG horseradish peroxidase-coupled secondary antibody. Detection was performed with an ECL system. Antibodies against IEX-1 (N-17), S-100, and GFAP, and anti-goat IgG HRP-coupled secondary antibodies were from Santa Cruz Biotechnology (Santa Cruz, CA). Anti-actin antibody was from Calbiochem (La Jolla, CA). Anti-mouse IgG HRP-coupled secondary antibody and ECL Western blot detection system were from Amersham Biosciences (Buckinghamshire, U.K.). As a result, IEX-1 expression was readily detectable after transfection with IEX-1 plasmid vector.

## Claims

1. Nucleic acid molecule encoding for IEX-1 polypeptide as a medicament.

2. Nucleic acid molecule according to claim 1, wherein the nucleic acid molecule encoding for IEX-1 polypeptide comprises the sequence of SEQ ID NO:1 or SEQ ID NO:2.

3. Nucleic acid molecule according to claim 1 or 2, wherein the nucleic acid molecule is a vector, in particular a viral vector derived from adenoviruses, adeno-associated virus, herpes viruses, retroviruses, or other mammalian viruses.

4. Nucleic acid molecule according to any of the preceding claims, wherein the expression of the nucleic acid molecule encoding for IEX-1 polypeptide is driven by a promoter, in particular by a promoter selected from SV40, CMV, thymidine kinase; E2A, LTR or wherein said promoter is an exogenously inducible promoter.

5. Nucleic acid molecule according to any of the preceding claims, wherein said medicament is formulated for a combined administration with a second therapy such as chemotherapy, radiation therapy, surgery, immune therapy, viral therapies or other gene therapeutic approaches relying on other lethal genes than IEX-1.

6. Nucleic acid molecule according to any of the preceding claims, wherein said medicament comprises an inhibitor of p53, in particular wherein said inhibitor is a nucleic acid molecule interfering with the expression of p53 or a nucleic acid molecule encoding for such a nucleic acid molecule interfering with the expression of p53, or where said inhibitor is any other molecule interfering with p53 expression, in particular wherein said molecule is pifithrin-α.

7. Use of a nucleic acid molecule encoding for IEX-1 polypeptide for the manufacture of a medicament for the treatment of gliomas.

8. Use according claim 7, wherein said gliomas are selected from ependymomas, astrocytomas, in particular glioblastoma, oligodendrogliomas, oligoastrocytomas, or mixed gliomas originating from at least two types of glial cells.

9. Use according to claim 7 or 8, wherein the nucleic acid molecule encoding for IEX-1 polypeptide comprises the sequence of SEQ ID NO:1 or SEQ ID NO:2.

10. Use according to any of claims 7 to 9, wherein the nucleic acid molecule is a vector, in particular a viral vector derived from adenoviruses, adeno-associated virus, herpes viruses, retroviruses, or other mammalian viruses.

11. Use according to any of claims 7 to 10, wherein the expression of the nucleic acid molecule encoding for IEX-1 polypeptide is driven by a promoter, in particular by a promoter selected from SV40, CMV, thymidine kinase, E2A, LTR or wherein said promoter is an exogenously inducible promoter.

12. Use according to any of claims 7 to 11, wherein said promoter confers a tissue specific expression of said IEX-1 polypeptide in glial cells or wherein said promoter confers a tumor specific expression.

13. Use according to any of claims 7 to 12, wherein said tissue specific promoter is the GFAP promoter or wherein the tumor specific promoter is the hTERT promotor.

14. Use according to any of claims 7 to 13, wherein said medicament is formulated for a combined administration with a second therapy such as chemotherapy, radiation therapy, surgery, immune therapy, viral therapies or other gene therapeutic approaches relying on other lethal genes than IEX-1.

15. Use according to any of claims 7 to 14, wherein said medicament comprises an inhibitor of p53, in particular wherein said inhibitor is a nucleic acid molecule interfering with the expression of p53 or a nucleic acid molecule encoding for such a nucleic acid molecule interfering with the expression of p53, or where said inhibitor is any other molecule interfering with p53 expression, in particular wherein said molecule is pifithrin-α.


### Annex to the application documents - subsequently filed sequences listing

SEQUENCE LISTING

<110>  TIMA Foundation

<120>  Use of IEX-1 for the treatment of glioma tumors

<130>  MAT-001 EP

<140>  EP 06 004 944
<141>  2006-03-10

<160>  6

<170>  PatentIn version 3.3

<210>  1
<211>  471
<212>  DNA
<213>  Homo sapiens

<400>  1
atgtgtcact ctcgcagctg ccacccgacc atgaccatcc tgcaggcccc gaccccggcc      60

ccctccacca tcccgggacc ccggcggggc tccggtcctg agatcttcac cttcgaccct     120

ctcccggagc ccgcagcggc ccctgccggg cgccccagcg cctctcgcgg gcaccgaaag     180

cgcagccgca gggttctcta ccctcgagtg gtccggcgcc agctgccagt cgaggaaccg     240

aacccagcca aaaggcttct ctttctgctg ctcaccatcg tcttctgcca gatcctgatg     300

gctgaagagg gtgtgccggc gcccctgcct ccagaggacg cccctaacgc cgcatccctg     360

gcgcccaccc ctgtgtcccc cgtcctcgag ccctttaatc tgacttcgga gccctcggac     420

tacgctctgg acctcagcac tttcctccag caacacccgg ccgccttcta a              471


<210>  2
<211>  582
<212>  DNA
<213>  Homo sapiens

<400>  2
atgtgtcact ctcgcagctg ccacccgacc atgaccatcc tgcaggcccc gaccccggcc      60

ccctccacca tcccgggacc ccggcggggc tccggtcctg agatcttcac cttcgaccct     120

ctcccggagc ccgcagcggc ccctgccggg cgccccagcg cctctcgcgg gcaccgaaag     180

cgcagccgca gggtctctct accctcgagt ggtgagtatc gccgaagtgg gcattcgcgg     240

tgtgcgctgc cctggagtca ctggggaacg acccgactcc agagcctcga cctgacctgt     300

ctcctgtttt gtctcccctt agtccggcgc cagctgccag tcgaggaacc gaacccagcc     360

aaaaggcttc tctttctgct gctcaccatc gtcttctgcc agatcctgat ggctgaagag     420

ggtgtgccgg cgcccctgcc tccagaggac gcccctaacg ccgcatccct ggcgcccacc     480

cctgtgtccc ccgtcctcga gccctttaat ctgacttcgg agccctcgga ctacgctctg     540

gacctcagca ctttcctcca gcaacacccg gccgccttct aa                        582

<210>  3
<211>  30
<212>  DNA
<213>  Artificial Sequence

<220>
<223>  Primer

<400>  3
cggaattccg atgtgtcact ctcgcagctg                                        30


<210>  4
<211>  30
<212>  DNA
<213>  Artificial Sequence

<220>
<223>  Primer

<400>  4
cggaattccg ttagaaggcg gccgggtgtt                                        30


<210>  5
<211>  65
<212>  DNA
<213>  Artificial Sequence

<220>
<223>  Oligonucleotide

<400>  5
gatccccgac tccagtggta atctacttca agagagtaga ttaccactgg agtctttttt      60

ggaaa                                                                   65


<210>  6
<211>  64
<212>  DNA
<213>  Artificial Sequence

<220>
<223>  Oligonucleotide

<400>  6
agcttttcca aaaagactcc agtggtaatc tactctcttg aagtagatta ccactggagt      60

cggg                                                                    64

SEQUENCE LISTING

<110>  TIMA Foundation

<120>  Use of IEX-1 for the treatment of glioma tumors

<130>  MAT-001 EP

<140>  unknown
<141>  2006-03-10

<160>  6

<170>  PatentIn version 3.3

<210>  1
<211>  471
<212>  DNA
<213>  Homo sapiens

<400>  1
atgtgtcact ctcgcagctg ccacccgacc atgaccatcc tgcaggcccc gaccccggcc      60

ccctccacca tcccgggacc ccggcggggc tccggtcctg agatcttcac cttcgaccct     120

ctcccggagc ccgcagcggc ccctgccggg cgccccagcg cctctcgcgg gcaccgaaag     180

cgcagccgca gggttctcta ccctcgagtg gtccggcgcc agctgccagt cgaggaaccg     240

aacccagcca aaaggcttct ctttctgctg ctcaccatcg tcttctgcca gatcctgatg     300

gctgaagagg gtgtgccggc gcccctgcct ccagaggacg cccctaacgc cgcatccctg     360

gcgcccaccc ctgtgtcccc cgtcctcgag ccctttaatc tgacttcgga gccctcggac     420

tacgctctgg acctcagcac tttcctccag caacacccgg ccgccttcta a              471


<210>  2
<211>  582
<212>  DNA
<213>  Homo sapiens

<400>  2
atgtgtcact ctcgcagctg ccacccgacc atgaccatcc tgcaggcccc gaccccggcc      60

ccctccacca tcccgggacc ccggcggggc tccggtcctg agatcttcac cttcgaccct     120

ctcccggagc ccgcagcggc ccctgccggg cgccccagcg cctctcgcgg gcaccgaaag     180

cgcagccgca gggtctctct accctcgagt ggtgagtatc gccgaagtgg gcattcgcgg     240

tgtgcgctgc cctggagtca ctggggaacg acccgactcc agagcctcga cctgacctgt     300

ctcctgtttt gtctcccctt agtccggcgc cagctgccag tcgaggaacc gaacccagcc     360

aaaaggcttc tctttctgct gctcaccatc gtcttctgcc agatcctgat ggctgaagag     420

ggtgtgccgg cgcccctgcc tccagaggac gcccctaacg ccgcatccct ggcgcccacc     480

cctgtgtccc ccgtcctcga gccctttaat ctgacttcgg agccctcgga ctacgctctg     540

gacctcagca ctttcctcca gcaacacccg gccgccttct aa                        582

<210> 3
<211> 30
<212> DNA
<213> Primer

<400> 3
cggaattccg atgtgtcact ctcgcagctg 30


<210> 4
<211> 30
<212> DNA
<213> Primer

<400> 4
cggaattccg ttagaaggcg gccgggtgtt 30


<210> 5
<211> 65
<212> DNA
<213> Oligonucleotide

<400> 5
gatccccgac tccagtggta atctacttca agagagtaga ttaccactgg agtctttttt 60

ggaaa 65


<210> 6
<211> 64
<212> DNA
<213> Oligonucleotide

<400> 6
agcttttcca aaaagactcc agtggtaatc tactctcttg aagtagatta ccactggagt 60

cggg 64